Europäisches Patentamt

European Patent Office

Office européen des brevets

(11) Veröffentlichungsnummer: **0 407 779 A1**

# EUROPÄISCHE PATENTANMELDUNG

(21) Anmeldenummer: **90111749.9**

(22) Anmeldetag: **21.06.90**

(51) Int. Cl.5: **A61B 17/22**, A61B 8/00

(30) Priorität: **10.07.89 DE 3922641**

(43) Veröffentlichungstag der Anmeldung:
**16.01.91 Patentblatt 91/03**

(84) Benannte Vertragsstaaten:
**BE DE FR GB NL**

(71) Anmelder: **Richard Wolf GmbH**
**Pforzheimer Strasse 32**
**D-7134 Knittlingen(DE)**

(72) Erfinder: **Belikan, Thomas, Dipl.-Ing.**
**Brahmsweg 4**
**D-7134 Knittlingen(DE)**
Erfinder: **Krauss, Werner, Dipl.-Ing. (FH)**
**Beethovenstrasse 84**
**D-7134 Knittlingen(DE)**

(74) Vertreter: **Wilcken, Thomas, Dipl.-Ing. et al**
**Musterbahn 1**
**D-2400 Lübeck(DE)**

(54) **Vorlaufstrecke für eine Lithotripsieeinrichtung.**

(57) Es ist eine diagnostische Vorlaufstrecke (1) für eine Lithotripsieeinrichtung mit einem Ultraschall-Stoßwellenwandler und mindestens einem Ultraschall-Ortungswandler beschrieben. Die Vorlaufstrecke bildet einen formstabilen Körper (1), der unter Formschluß lösbar auf dem Ultraschall-Ortungswandler (2) angeordnet ist. Die Vorlaufstrecke weist einen Wellenwiderstand auf, der im wesentlichen demjenigen von Gewebe entspricht.

FIG. 5

EP 0 407 779 A1

## VORLAUFSTRECKE FÜR EINE LITHOTRIPSIEEINRICHTUNG

Die Erfindung betrifft eine Vorlaufstrecke für eine Lithotripsieeinrichtung mit einem Ultraschall-Stoßwellenwandler und mindestens einem Ultraschall-Ortungswandler.

Es ist bekannt, die Ortung eines mittels einer Lithotripsieeinrichtung zu zerstörenden Objektes, beispielsweise eines Konkrementes, im Körper eines zu behandelnden Patienten mittels Ultraschallwellen vorzunehmen. Hierfür wird ein Ultraschall-Ortungswandler, beispielsweise ein B-Scanner, verwendet.

Die Qualität der Ortung mit Hilfe eines Ultraschall-Ortungswandlers ist im allgemeinen zufriedenstellend, wenn der Schallkopf des Ultraschall-Ortungswandlers direkt an der Haut des Patienten anliegt. Sofern dies jedoch nicht der Fall ist, wird die Ortung des zu zerstörenden Objektes erheblich dadurch erschwert, daß Mehrfachechos auftreten und aufgrund der schlechten akusti schen Anpassung von Hautoberfläche und Schallkopfoberfläche des Ortungswandlers viele Ultraschallwellen an der Haut so reflektiert werden, daß sie nicht mehr auf den Schallkopf auftreffen. Hierdurch ergibt sich eine erhebliche Verschlechterung des aus den Empfangssignalen des Ultraschall-Ortungswandlers gewonnenen Bildes.

Dieses Problem tritt insbesondere auf bei Lithotripsieeinrichtungen, bei denen der Ultraschall-Ortungswandler im Ultraschall-Stoßwellenwandler angeordnet ist. Beispielhaft sei hier auf einige Ausführungsbeispiele der DE-OS 35 43 867 hingewiesen.

Bedingt durch den von dem Ultraschall-Stoßwellenwandler ausgehenden Stoßwellenkegel ist es hierbei nicht möglich, mit dem Schallkopf des Ortungswandlers nahe an den Fokus des Stoßwellenwandlers heranzufahren, da andernfalls zuviel Stoßwellenenergie vom Schallkopf des Ortungswandlers abgeschattet würde. Insbesondere bei der Ortung von oberflächlichen Strukturen wie Gallenblasensteinen, Speichelsteinen oder oberflächennahen Tumoren kommt es infolgedessen zu der weiter oben beschriebenen Verschlechterung der Qualität des gewonnenen Ortungsbildes. Darüber hinaus ist der Abstand vom Schallkopf des Ortungswandlers zum Körper des Patienten abhängig von dessen Körperbau. So wird der Abstand bei Kindern und schlanken Personen größer sein als bei korpulenten Patienten.

Der Erfindung liegt vor dem aufgezeigten Hintergrund nun die Aufgabe zugrunde, hier Abhilfe zu schaffen. Insbesondere soll also eine Vorrichtung geschaffen werden, die bei bekannten Lithotripsieeinrichtungen stets eine gute Ultraschallortung des zu zerstörenden Objektes im Patientenkörper gewährleistet, ohne dabei therapeutische Ultra schall-Stoßwellen abzuschatten.

Diese Aufgabe wird gelöst durch eine diagnostische Vorlaufstrecke mit den Merkmalen des Anspruches 1.

Die erfindungsgemäße Vorlaufstrecke ist zwischen dem Schallkopf des Ortungswandlers und der Haut des Patienten fixierbar. Hierdurch wird der Abstand vom Schallkopf zur Haut überbrückt. Weiter oben beschriebene Mehrfachechos treten demgemäß nicht auf. Dadurch, daß der Wellenwiderstand der Vorlaufstrecke im wesentlichen jenem vom Gewebe entspricht, wird eine hervorragende Ankopplung der vom Ortungswandler ausgesandten Ultraschallwellen an den Patientenkörper gewährleistet. Durch den Formschluß zwischen dem Ortungswandler und der Vorlaufstrecke werden etwaige Luftblaseneinschlüsse vermieden, die eine Beeinträchtigung der Qualität des Ortungsbildes zur Folge hätten. Im übrigen können therapeutische Ultraschall-Stoßwellen aufgrund der akustischen Eigenschaft der Vorlaufstrecke weitgehend ungehindert durch sie hindurchtreten.

Der Formschluß zwischen der Vorlaufstrecke und dem Ortungswandler kann vorteilhaft dadurch verbessert werden, daß die Vorlaufstrecke zum Ortungswandler hin komplementär zu dessen Oberflächengeometrie ausgebildet ist. Hierdurch ergibt sich eine verlustfreie Ankoppelung der Ultraschallwellen an den Patientenkörper.

Ein sicherer Sitz der Vorlaufstrecke auf dem Ortungswandler kann dadurch erreicht werden, daß sie weit über diesen greift. In dem Falle, in dem der Ortungswandler ein B-Scanner herkömmlicher Bauart ist,bedeutet dies, daß die Vorlaufstrecke weit über den Schaft des B-Scanners greift.

Die mechanische Festigkeit der Vorlaufstrecke kann dadurch erhöht werden, daß sie im Bereich ihres auf dem Ortungswandler sitzenden Endes mit einer Gewebeeinlage versehen ist.

Die Stabilität des Sitzes auf dem Ortungswandler kann auch dadurch erhöht werden, daß die Vorlaufstrecke im Bereich ihres auf dem Ortungswandler sitzenden Endes eine Materialverstärkung aufweist. Diese Materialverstärkung kann rein geometrischer Natur sein. Es ist aber auch an eine Verstärkung durch eine entsprechende Materialeigenschaft gedacht.

Im übrigen ist es auch möglich, die Vorlaufstrecke auf dem Ortungswandler dadurch zu fixieren, daß sie mittels einer Manschette an ihrem auf dem Ortungswandler sitzenden Ende praktisch festgeschnallt wird.

Als Material für die Vorlaufstrecke eignet sich in hervorragender Weise ein Hydro-Gel. Insbesondere kann sie aus Polyacrylamid-Agargel bestehen, welches praktisch dieselben akustischen Eigenschaften wie Wasser aufweist. Die Vorlaufstrecke aus Polyacrylamid-Agargel kann sich auch ohne zusätzliches Koppelgel optimal an die Haut des Patienten und an den Schallkopf des Ortungswandlers ohne Lufteinschlüsse anlegen.

Die Erfindung wird nachfolgend anhand einiger Ausführungsbeispiele gemäß den Zeichnungen erläutert. Hierbei zeigt:

Figur 1 einen Ultraschall-Ortungswandler mit auf ihm sitzender diagnostischer Vorlaufstrecke,

Figur 2a eine Ansicht der Vorlaufstrecke, die auf einem Ortungswandler mittels einer Manschette befestigt ist,

Figur 2b eine Schnittansicht der Anordnung gemäß Figur 2a,

Figur 3 eine Vorlaufstrecke auf einem Ortungswandler, wobei die Stabilität ihres Sitzes mit einer Materialverstärkung erfolgt,

Figur 4 eine Vorlaufstrecke mit einer Gewebeeinlage zur Erhöhung ihrer Festigkeit, und

Figur 5 ein schematisches Anwendungsbeispiel der Vorlaufstrecke.

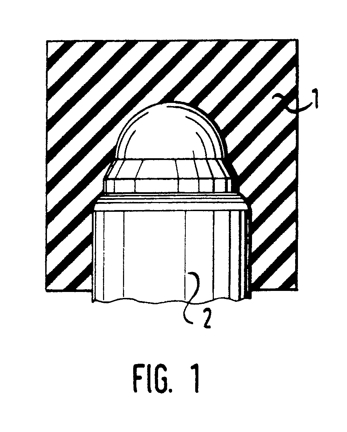

Figur 1 zeigt eine Vorlaufstrecke 1, die auf einen Ultraschall-Ortungswandler 2 gesetzt ist. Man erkennt deutlich, daß die Vorlaufstrecke 1 unter Formschluß auf dem Ortungswandler 2 sitzt. Die Vorlaufstrecke 1 bildet einen formstabilen Körper, dessen Wellenwiderstand im wesentlichen jenem vom Gewebe entspricht. Im gezeigten Ausführungsbeispiel ist die Vorlaufstrecke 1 zum Ortungswandler 2 hin komplementär zu dessen Oberflächengeometrie ausgebildet. Hierdurch wird eine verlustfreie Ankopplung der vom Ortungswandler 2 ausgehenden Ultraschallwellen an den Patientenkörper erzielt, und zwar ohne Einschluß von etwaigen Luftbläschen.

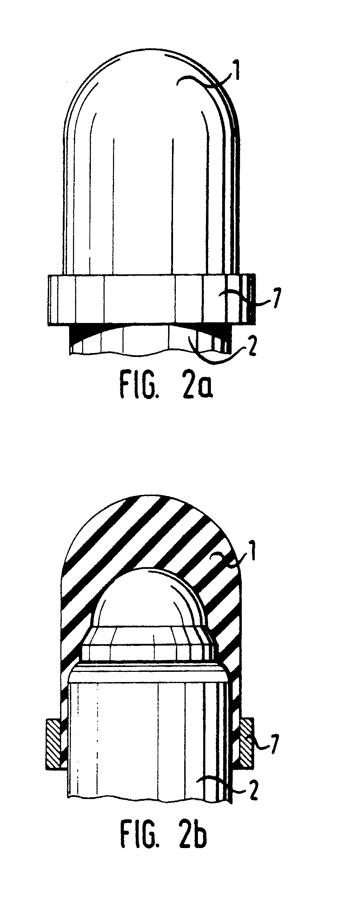

Figur 2a zeigt eine Vorlaufstrecke 1 auf dem Schaft eines Ortungswandlers 2 sitzend, wobei die Vorlaufstrecke 1 mittels einer um sie greifenden Manschette 7 am Ortungswandler 2 befestigt ist. Einzelheiten ergeben sich aus der Figur 2b, in der eine Schnittansicht der Ansicht aus Figur 2a gezeigt ist. Deutlich erkennbar ist darüber hinaus, daß die Vorlaufstrecke 1 relativ weit über den Ortungswandler 2 greift. An ihrem auf dem Ortungswandler 2 sitzenden Ende ist die Manschette 7 vorgesehen, mittels der die Vorlaufstrecke 1 auf dem Ortungswandler 2 fixiert ist.

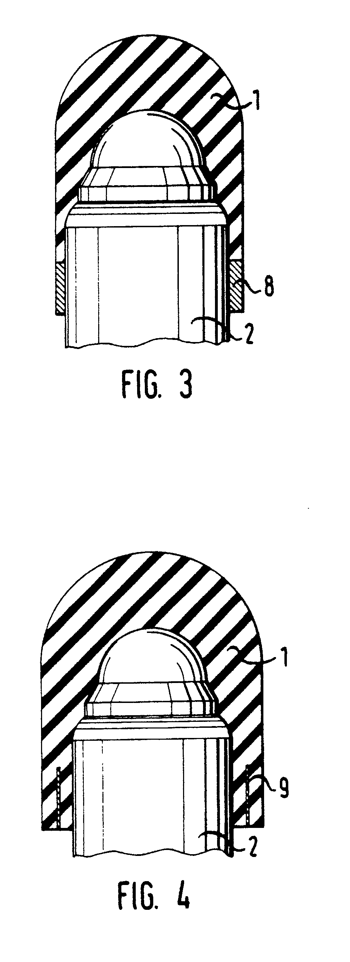

Figur 3 zeigt demgegenüber, wie der Sitz der Vorlaufstrecke 1 auf dem Ortungswandler 2 gemäß dieses Ausführungsbeispieles mittels einer Materialverstärkung im Bereich ihres auf dem Ortungswandler 2 sitzenden Endes gesichert ist. Wenn die Vorlaufstrecke 1 beispielsweise aus Polyacrylamid-Agargel besteht, kann diese Materialverstärkung 8 dadurch erreicht werden, daß der untere Rand der Vorlaufstrecke wesentlich stärker vernetzt und somit relativ hart ist. Eine derartige Verhärtung kann erreicht werden durch eine Abänderung des Monomer-Wasser-Verhältnisses.

Maßnahmen wie in den Figuren 2a, 2b und 3 dargestellt sind in den meisten Fällen angebracht, da der Schwerpunkt der Vorlaufstrecke 1 je nach Dicke und Länge deutlich oberhalb des Ortungswandlers 2 liegen wird und da in vielen Fällen der Ortungswandler 2, sofern er in dem Ultraschall-Stoßwellenwandler der Lithotripsieeinheit eingebaut ist, zumeist nicht senkrecht in diesem angeordnet ist.

In Figur 4 ist eine Vorlaufstrecke 1 auf einem Ortungswandler 2 sitzend dargestellt, wobei im Bereich ihres auf dem Ortungswandler 2 sitzenden Endes eine Gewebeeinlage 9 eingearbeitet ist. Diese Gewebeeinlage 9 erhöht die Festigkeit der Vorlaufstrecke 1 und kann somit auch zu einem sicheren Sitz auf dem Ortungswandler 2 beitragen.

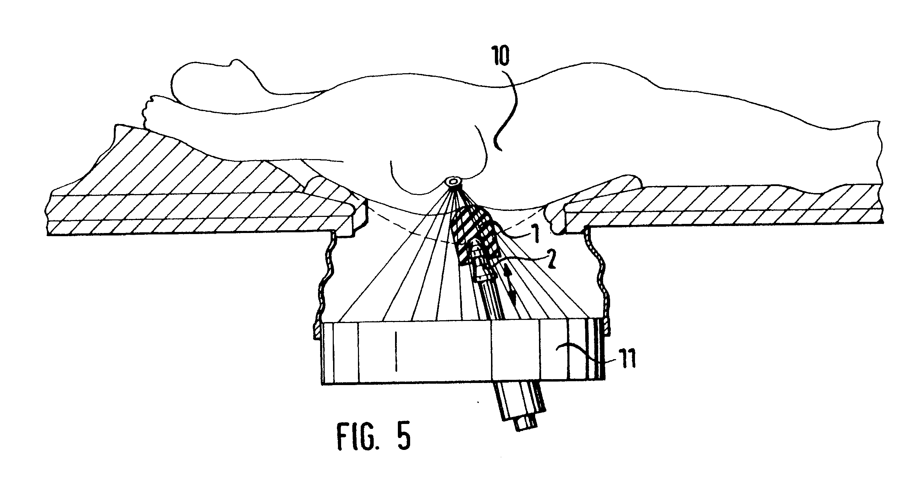

Figur 5 zeigt ein Anwendungsbeispiel der Vorlaufstrecke 1. In den Ultraschall-Stoßwellenwandler 11 der Lithotripsieeinrichtung ist ein Ultraschall-Ortungswandler 2 eingebaut, und zwar derart,daß er in Richtung des doppelten Pfeiles verfahrbar ist. Die Vorlaufstrecke 1 ist in der beschriebenen Weise auf dem Ortungswandler 2 angeordnet. Sie überbrückt die Strecke zwischen dem Ortungswandler 2 und einem Patientenkörper 10. Bei einem korpulenten Patienten (schematisch mit der gestrichelten Linie angedeutet) ist der Abstand zwischen dem Schallkopf des Ortungswandlers 2 und der Haut des Patienten geringer als bei einem schlanken Patienten oder einem Kind. Im Extremfall berührt die Haut des Patienten den Schallkopf des Ortungswandlers 2 direkt, so daß es einer Zwischenschaltung der erfindungsgemäßen Vorlaufstrecke 1 nicht bedarf. Dies wird freilich nur in den seltensten Fällen eintreten.

**Ansprüche**

1. Vorlaufstrecke für eine Lithotripsieeinrichtung mit einem Ultraschall-Stoßwellenwandler und mindestens einem Ultraschall-Ortungswandler, dadurch gekennzeichnet, daß sie einen formstabilen Körper (1) bildet, der unter Formschluß lösbar auf dem Ultraschall-Ortungswandler (2) angeordnet ist und einen Wellenwiderstand aufweist, der im wesentlichen jenem von Gewebe entspricht.

2. Vorlaufstrecke nach Anspruch 1, dadurch gekennzeichnet, daß sie zur verlustfreien Ankopplung der vom Ultraschall-Ortungswandler (2) ausgehenden Ultraschallwellen an den Patientenkörper (10) zum Ultraschall-Ortungswandler (2) hin komplementär zu dessen Oberflächengeometrie ausgebildet ist.

3. Vorlaufstrecke nach einem der Ansprüche 1 oder 2, dadurch gekennzeichnet, daß sie so weit über den Ultraschall-Ortungswandler (2) greift, daß ein sicherer Sitz auf diesem gewährleistet ist.

4. Vorlaufstrecke nach einem der Ansprüche 1 bis 3, dadurch gekennzeichnet, daß sie im Bereich ihres auf dem Ultraschall-Ortungswandler (2) sitzenden Endes eine Gewebeeinlage (9) zur Erhöhung ihrer Festigkeit aufweist.

5. Vorlaufstrecke nach einem der Ansprüche 1 bis 4, dadurch gekennzeichnet, daß sie im Bereich ihres auf dem Ultraschall-Ortungswandler (2) sitzenden Endes eine Materialverstärkung (8) zur Sicherung ihres Sitzes auf dem Ultraschall-Ortungswandler (2) aufweist.

6. Vorlaufstrecke nach einem der Ansprüche 1 bis 5, dadurch gekennzeichnet, daß sie mittels einer Manschette (7) an ihrem auf dem Ultraschall-Ortungswandler (2) sitzenden Ende an dessen Schaft fixierbar ist.

7. Vorlaufstrecke nach einem der Ansprüche 1 bis 6, dadurch gekennzeichnet, daß sie aus einem Hydrogel besteht.

8. Vorlaufstrecke nach Anspruch 7, dadurch gekennzeichnet, daß sie aus Polyacrylamid-Agargel besteht.

FIG. 1

FIG. 2a

FIG. 2b

FIG. 3

FIG. 4

FIG. 5

Europäisches
Patentamt

**EUROPÄISCHER RECHERCHENBERICHT**

Nummer der Anmeldung

EP 90 11 1749

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-2 003 701 (BLOOMER) <br> * Seite 1, Zeilen 42-71,102-120; Fig. * <br> --- | 1-8 | A 61 B 17/22 <br> A 61 B 8/00 |
| X | GB-A-2 009 563 (EMI) <br> * Seite 1, Zeilen 77-104; Figur 1 * <br> --- | 1-6 | |
| A,D | FR-A-2 591 467 (WOLF) <br> * Seite 5, Zeile 33 - Seite 6, Zeile 25; Figur 1 * <br> --- | 1 | |
| A | EP-A-0 256 202 (SIEMENS) <br> * Spalte 2, Zeile 45 - Spalte 3, Zeile 4; Figur 1 * <br> --- | 1 | |
| X,P | EP-A-0 344 773 (TAKIRON) <br> * Seite 11, Zeilen 32-40; Seite 12, Zeilen 2-5; Figuren 1-9 * <br> ----- | 1-6 | |

|   |
|---|
| **RECHERCHIERTE SACHGEBIETE (Int. Cl.5)** |
| A 61 B |

Der vorliegende Recherchenbericht wurde für alle Patentansprüche erstellt

| Recherchenort | Abschlußdatum der Recherche | Prüfer |
|---|---|---|
| DEN HAAG | 15-10-1990 | MOERS R.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

........................................................
& : Mitglied der gleichen Patentfamilie, übereinstimmendes Dokument

EPO FORM 1503 03.82 (P0403)